# EUROPÄISCHE PATENTSCHRIFT

(11) **EP 0 658 165 B1**
(45) Veröffentlichungstag und Bekanntmachung des Hinweises auf die Patenterteilung: **26.05.1999**
(21) Anmeldenummer: 93919184.7
(22) Anmeldetag: 26.08.1993
(51) Int. Cl.: C07H 15/04, C07C 233/18, B01F 17/00, B01F 17/56, B01F 17/22, D06P 1/48, D06P 1/649, C09B 67/46

(54) **Verwendung von Dispergiermitteln zur Herstellung von Farbstoff- und Pigmentpräparationen**
Use of dispersing agents for the preparation of compositions containing dyes and pigments
Utilisation d'agents dispersants pour la préparation de compositions contenant des colorants et pigments

(30) Priorität: 03.09.1992 DE 4229442
(43) Veröffentlichungstag der Anmeldung: 21.06.1995
(73) Patentinhaber: Henkel Kommanditgesellschaft auf Aktien, 40191 Düsseldorf (DE)
(72) Erfinder: ULLRICH, Claudia, D-40211 Düsseldorf (DE); WEGENER, Ingo, D-40589 Düsseldorf (DE); FABRY, Bernd, D-41352 Korschenbroich (DE)
(86) Internationale Anmeldenummer: EP9302302
(87) Internationale Veröffentlichungsnummer: WO9405680

(56) Entgegenhaltungen:
- EP-A- 0 301 298
- EP-A- 0 497 172
- EP-A- 0 530 708
- WO-A-90/03977
- WO-A-92/06156
- WO-A-92/06160
- WO-A-92/06172
- WO-A-92/06984
- US-A- 1 985 424
- US-A- 2 703 798
- DATABASE WPI Derwent Publications Ltd., London, GB; AN 92-020327 & JP,A,3 269 097 (KAO CORP.) 29. November 1991

## Beschreibung

### Gebiet der Erfindung

Die Erfindung betrifft die Verwendung von Dispergiermitteln mit einem Gehalt an Alk(en)ylglykosiden und/oder Polyhydroxyfettsäureamiden zusammen mit weiteren Tensiden und/oder Polymeren zur Herstellung von Farbstoff- und Pigmentpräparationen.

### Stand der Technik

Farbstoffzubereitungen oder Färbebäder enthalten üblicherweise als Dispergiermittel Ligninsulfonate oder Sulfonate von Kondensationsprodukten des Formaldehyds mit Naphthalin oder Ketonen [**DE-A-40 18 873, DE-A-17 20 729**]. Nach dem bestimmungsgemäßen Gebrauch gelangen die genannten Produkte ins Abwasser und sind daraus in Kläranlagen nur zu einem geringen Teil biologisch abbaubar bzw. eliminierbar. In der Vergangenheit hat es nicht an Versuchen gefehlt, Dispergiermittel mit verbesserten ökologischen Eigenschaften zu entwickeln. In der **DE-A-40 22 220** (Bayer) wird beispielsweise vorgeschlagen, Mischungen von Ketonen und nichtionischen Tensiden als Färbereihilfsmittel einzusetzen. Aus der **EP-A-0 463 401** (BASF) sind Dispergiermittel bekannt, die Kombinationen von Arylsulfonsäure-Formaldehyd-Kondensaten und langkettigen aliphatischen Carbonsäuren enthalten. Die genannten Stoffe weisen zwar gegenüber dem Stand der Technik eine verbesserte biologische Abbaubarkeit auf, die Anforderungen hinsichtlich eines zumindest vergleichbar guten Dispergiervermögens werden jedoch in aller Regel nicht erfüllt. Aus der **EP-A 0 530 708** (Albright & Wilson) ist schließlich die Herstellung wäßriger, konzentrierter Suspensionen unterschiedlichster Stoffe bekannt, wobei als Dispergatoren auch Alkyloligoglucoside eingesetzt werden.

Die Aufgabe der Erfindung bestand somit darin, neue Dispergiermittel zu entwickeln, die frei von den geschilderten Nachteilen sind.

### Beschreibung der Erfindung

Gegenstand der Erfindung ist die Verwendung von Dispergiermitteln, enthaltend
(a) Alkyl- und/oder Alkenyloligoglykoside der Formel **(I)**,

   **R**^{**1**}**O-[G]**_{**p**} (I)

   in der R¹ für einen Alkyl- und/oder Alkenylrest mit 6 bis 22 Kohlenstoffatomen, [G] für einen Zuckerrest mit 5 oder 6 Kohlenstoffatomen und p für eine Zahl von 1 bis 10 steht, und/oder
(b) Polyhydroxyfettsäureamide der Formel **(II)**, 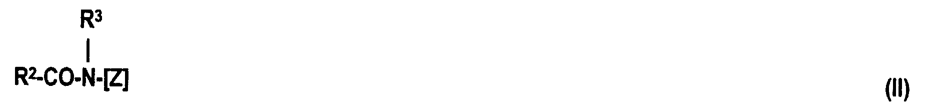 in der R²CO für einen aliphatischen Acylrest mit 6 bis 22 Kohlenstoffatomen, R³ für einen Alkyl- oder Hydroxyalkylrest mit 1 bis 4 Kohlenstoffatomen und [Z] für einen linearen oder verzweigten Polyhydroxyalkylrest mit 3 bis 10 Kohlenstoffatomen und 3 bis 10 Hydroxylgruppen steht, sowie gegebenenfalls
(c) weitere anionische, nichtionische, amphotere bzw. zwitterionische Tenside und/oder Polymere
zur Herstellung von Farbstoff- und Pigmentpräparationen.

Überraschenderweise wurde gefunden, daß Alk(en)yloligoglycoside und/oder Polyhydroxyfettsäureamide, insbesondere Fettsäure-N-alkyl-glucamide, in Kombination mit weiteren Tensiden und/oder Polymeren die Bildung feinteiliger, homogener Feststoffdispersionen unterstützen. Ein weiterer Vorteil der neuen Mittel besteht in ihrer vollständigen biologischen Abbaubarkeit.

### Alkyl- und/oder Alkenyloligoglykoside

Alkyl- und Alkenyloligoglykoside stellen bekannte Stoffe dar, die nach den einschlägigen Verfahren der präparativen organischen Chemie erhalten werden können. Stellvertretend für das umfangreiche Schrifttum sei hier auf die Schriften **EP-A1-0 301 298** und **WO 90/3977** verwiesen. Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligo**glucoside**.

Die Indexzahl p in der allgemeinen Formel **(I)** gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt.

Der Alkyl- bzw. Alkenylrest R¹ kann sich von primären Alkoholen mit 6 bis 11, vorzugsweise 8 bis 10 Kohlenstoffatomen ableiten. Typische Beispiele sind Capronalkohol, Caprylalkohol, Caprinalkohol und Undecylalkohol sowie deren technische Mischungen, wie sie beispielsweise bei der Hydrierung von technischen Fettsäuremethylestem oder im Verlauf der Hydrierung von Aldehyden aus der Roelen'schen Oxosynthese anfallen. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R¹ kann sich femer auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.

### Polyhydroxyfettsäuramide

Auch bei den Polyhydroxyfettsäureamiden handelt es sich um bekannte Stoffe, die üblicherweise durch reduktive Aminierung eines reduzierenden Zuckers mit einem Alkylamin oder einem Alkanolamin und nachfolgende Acylierung mit einer Fettsäure, einem Fettsäurealkylester oder einem Fettsäurechlorid erhalten werden können. Hinsichtlich der Verfahren zu ihrer Herstellung sei auf die US-Patentschriften **US 1,985,424**, **US 2,016,962** und **US 2,703,798** sowie die Intemationale Patentanmeldung **WO 92/06984** verwiesen. Vorzugsweise leiten sich die Polyhydroxyfettsäureamide von reduzierenden Zuckem mit 5 oder 6 Kohlenstoffatomen, insbesondere von der Glucose ab. Die bevorzugten Polyhydroxyfettsäureamide stellen daher **Fettsäure-N-alkylglucamide** dar, wie sie durch die Formel **(III)** wiedergegeben werden:

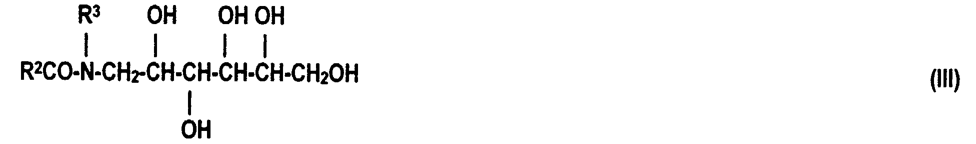

Vorzugsweise werden als Polyhydroxyfettsäureamide Fettsäure-N-alkylglucamide der Formel **(III)** eingesetzt, in der R³ für eine Alkylgruppe steht und R²CO für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. derer technischer Mischungen steht. Besonders bevorzugt sind Fettsäure-N-alkylglucamide der Formel **(III)**, die durch reduktive Aminierung von Glucose mit Methylamin und anschließende Acylierung mit Laurinsäure oder C_{12/14}-Kokosfettsäure bzw. einem entsprechenden Derivat erhalten werden.

### Tenside

Typische Beispiele für Tenside, die im Sinne der Erfindung als Komponente (c) eingesetzt werden können sind **anionische Tenside** vom Typ der Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglyrid(ether)sulfate, Fettsäureamid(ether)sulfate, Sulfosuccinate, Sulfosuccinamate, Sulfotriglyceride, Ethercarbonsäuren, Alkyl-oligoglucosidsulfate und Alkyl(ether)phosphate. Sofem die anionischen Tenside Polyglycoletherketten enthalten, können sie eine konventionelle, vorzugsweise jedoch eine eingeengte Homologenverteilung aufweisen. Typische Beispiele für **nichtionische Tenside** sind Fettalkoholpolyglycolether, Alkylphenolpolyglycolether, Fettsäurepolyglycolester, Fettsäureamidpolygylcolether, Fettaminpolyglycolether, alkoxylierte Triglyceride, Polyolfettsäureester, Zuckerester, Sorbitanester und Polysorbate. Sofern die nichtionischen Tenside Polyglycoletherketten enthalten, können sie eine konventionelle, vorzugsweise jedoch eine eingeengte Homologenverteilung aufweisen. Typische Beispiele für **amphotere bzw. zwitterionische Tenside** sind Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S.54-124** oder **J.Falbe (ed.), "Katalysatoren, Tenside und Mineralöladditive", Thieme Verlag, Stuttgart, 1978, S.123-217** verwiesen.

### Polymere und weitere Zusatzstoffe

Typische Beispiele für Polymere, die im Sinne der Erfindung ebenfalls als Komponente (c) in Frage kommen, sind gegebenenfalls sulfonierte Kondensate des Formaldehyds mit Phenol oder Naphthalin. Als weitere Zusatzstoffe können zusammen mit den Detergensgemischen bis zu 5 Gew.-% - bezogen auf die Mittel - weitere Dispergatoren, Entschäumer, Hydrotrope, Stellmittel, Komplexierungsmittel und Biozide eingesetzt werden.

### Beispiele

Das Dispergiervermögen der erfindungsgemäßen Mittel wurde an Hand der Filtration verschiedener Farbstoffdispersionen durch ein Papierfilter (AATCC-Test) ermittelt. Hierbei wird eine Dispersion mittels Vakuum durch zwei Papierfilter gesaugt und die Menge und Verteilung des Filterrückstandes auf dem getrockneten Filterpapier visuell gegen einen Maßstab bestimmt. Gleichzeitig wird auch die Filtrationszeit gemessen. Der Test gibt Aufschluß über den Zustand der Dispersion im Hinblick auf die Feinheit der Partikel. Zu Einzelheiten sei auf eine Übersicht in **Melliand Textilber. 10, 755 (1988)** verwiesen. In 500 ml entionisiertem Wasser wurden bei 80°C 1,5 g des Dispergiermittels - bezogen auf Feststoffgehalt - gelöst und mit Essigsäure auf pH 5 eingestellt. Unter Umrühren wurden der Lösung 0,3 g des Farbstoffs Palanilscharlach RR zugesetzt. Die Flotte wurde mit einer Geschwindigkeit von 3°C/min auf 120°C erhitzt und bei dieser Temperatur 30 min gehalten. Anschließend ließ man auf 80°C abkühlen und filtrierte durch eine Lage von zwei Rundfilterpapieren (oben MN 640 d, unten Blauband 589.3). Beurteilt wurde die Filtrationszeit sowie die Homogenität des Farbstoffverlaufs und des Rückstandes auf dem oberen Filterpapier: (I) = homogen, keine Stippen, (II) = homogen, wenig Stippen, (III) = wenig homogen, einige Stippen und (IV) = inhomogen. Die Ergebnisse sind in Tabelle 1 zusammengefaßt. Die Mischungen 1 bis 7 sind erfindungsgemäß, die Mischungen V1 und V2 dienen zum Vergleich.

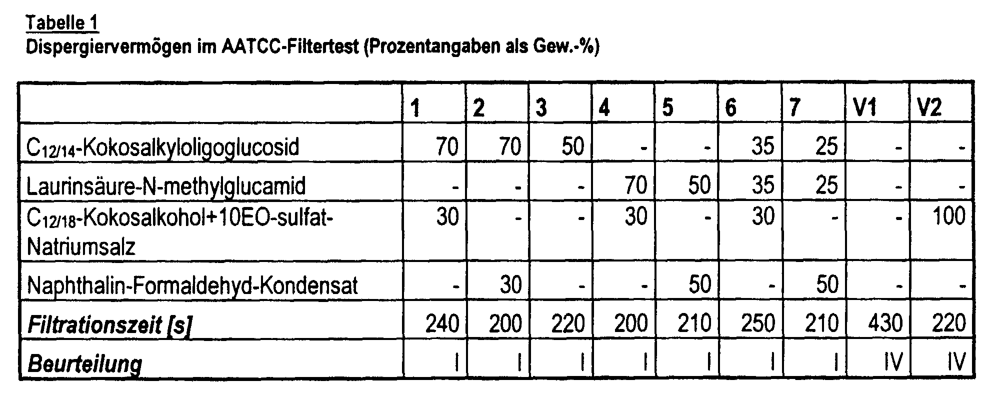

## Patentansprüche

1. Verwendung von Dispergiermitteln, enthaltend
(a) Alkyl- und/oder Alkenyloligoglykoside der Formel (I),
**R**^{**1**}**O-[G]**_{**p**} (I)
in der R¹ für einen Alkyl- und/oder Alkenylrest mit 6 bis 22 Kohlenstoffatomen, [G] für einen Zuckerrest mit 5 oder 6 Kohlenstoffatomen und p für eine Zahl von 1 bis 10 steht, und/oder
(b) Polyhydroxyfettsäureamide der Formel **(II)**, 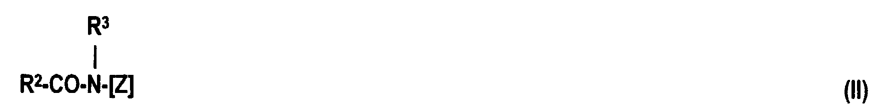 in der R²CO für einen aliphatischen Acylrest mit 6 bis 22 Kohlenstoffatomen, R³ für einen Alkyl- oder Hydroxyalkylrest mit 1 bis 4 Kohlenstoffatomen und [Z] für einen linearen oder verzweigten Polyhydroxyalkylrest mit 3 bis 10 Kohlenstoffatomen und 3 bis 10 Hydroxylgruppen steht, sowie gegebenenfalls
(c) weitere anionische, nichtionische, amphotere bzw. zwitterionische Tenside und/oder Polymere
zur Herstellung von Farbstoff- und Pigmentpräparationen.

2. Verwendung von Dispergiermitteln nach Anspruch 1, **dadurch gekennzeichnet**, daß man anionische Tenside einsetzt, die ausgewählt sind aus der Gruppe, die von Alkylbenzolsulfonaten, Alkansulfonaten, Olefinsulfonaten, Alkylethersulfonaten, Glycerinethersulfonaten, α-Methylestersulfonaten, Sulfofettsäuren, Alkylsulfaten, Fettalkoholethersulfaten, Glycerinethersulfaten, Hydroxymischethersulfaten, Monoglycerid(ether)sulfaten, Fettsäureamid(ether)sulfaten, Sulfosuccinaten, Sulfosuccinamaten, Sulfotriglyceriden, Ethercarbonsäuren, Alkyloligoglucosidsulfaten und Alkyl(ether)phosphaten gebildet wird.

3. Verwendung von Dispergiermitteln nach den Ansprüchen 1 und 2, **dadurch gekennzeichnet**, daß man nichtionische Tenside einsetzt, die ausgewählt sind aus der Gruppe, die von Fettalkoholpolyglycolethern, Alkylphenolpolyglycolethern, Fettsäurepolyglycolestern, Fettsäureamidpolyglycolethem, Fettaminpolyglycolethem, alkoxylierten Triglyceriden, Polyolfettsäureestern, Zuckerestem, Sorbitanestem und Polysorbaten gebildet wird.

4. Verwendung von Dispergiermitteln nach den Ansprüchen 1 bis 3, **dadurch gekennzeichnet**, daß man amphotere bzw. zwitterionische Tenside einsetzt, die ausgewählt sind aus der Gruppe, die von Alkylamidobetainen, Aminopropionaten, Aminoglycinaten, Imidazoliniumbetainen und Sulfobetainen gebildet wird.

5. Verwendung von Dispergiermitteln nach den Ansprüchen 1 bis 4, **dadurch gekennzeichnet**, daß man weiterhin sulfonierte Kondensate des Formaldehyds mit Phenol oder Naphthalin einsetzt.

## Claims

1. The use of dispersants containing
(a) alkyl and/or alkenyl oligoglycosides corresponding to formula **(I)**:
**R**^{**1**}**O-[G]**_{**p**} (I)
in which R¹ is an alkyl and/or alkenyl radical containing 6 to 22 carbon atoms, [G] is a sugar unit containing 5 or 6 carbon atoms and p is a number of 1 to 10,
and/or
(b) polyhydroxy fatty acid amides corresponding to formula **(II)**: 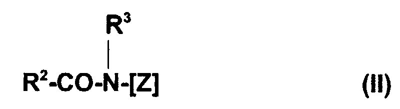 in which R²CO is an aliphatic acyl radical containing 6 to 22 carbon atoms, R³ is hydrogen, an alkyl or hydroxyalkyl radical containing 1 to 4 carbon atoms and [Z] is a linear or branched polyhydroxyalkyl radical containing 3 to 10 carbon atoms and 3 to 10 hydroxyl groups,
and optionally
(c) other anionic, nonionic, amphoteric or zwitterionic surfactants and/or polymers,
for the production of dye and pigment preparations.

2. The use of dispersants as claimed in claim 1, **characterized in that** anionic surfactants selected from the group consisting of alkyl benzene sulfonates, alkane sulfonates, olefin sulfonates, alkyl ether sulfonates, glycerol ether sulfonates, α-methyl ester sulfonates, sulfofatty acids, alkyl sulfates, fatty alcohol ether sulfates, glycerol ether sulfates, hydroxy mixed ether sulfates, monoglyceride (ether) sulfates, fatty acid amide (ether) sulfates, sulfosuccinates, sulfosuccinamates, sulfotriglycerides, ether carboxylic acids, alkyl oligoglucoside sulfates and alkyl (ether) phosphates are used.

3. The use of dispersants as claimed in claim 1, **characterized in that** nonionic surfactants selected from the group consisting of fatty alcohol polyglycol ethers, alkylphenol polyglycol ethers, fatty acid polyglycol esters, fatty acid amide polyglycol ethers, fatty amine polyglycol ethers, alkoxylated triglycerides, polyol fatty acid esters, sugar esters, sorbitan esters and polysorbates are used.

4. The use of dispersants as claimed in claims 1 to 3, **characterized in that** amphoteric or zwitterionic surfactants selected from the group consisting of alkyl amidobetaines, aminopropionates, aminoglycinates, imidazolinium betaines and sulfobetaines are used.

5. The use of dispersants as claimed in claims 1 to 4, **characterized in that** optionally sulfonated condensates of formaldehyde with phenol or naphthalene are additionally used.

## Revendications

1. Utilisation d'agents dispersants renfermant :
a) des alkyl- et/ou alkényloligoglycosides de formule (I)
**R**^{**1**}**O-[G]**_{**p**} (I)
dans laquelle R¹ représente un radical alkyle- et/ou alkényle ayant de 6 à 22 atomes de carbone.
[G] représente un radical de sucre ayant 5 ou 6 atomes de carbone et p représente un nombre allant de 1 à 10.
b) des amides d'acide gras polyhydroxylés de formule (II) 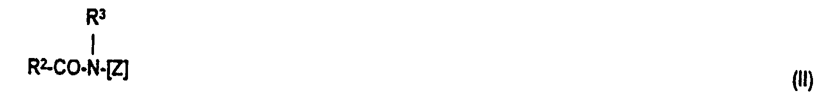 dans laquelle R²CO représente un reste acyle aliphatique ayant de 6 à 22 atomes de carbone, R³ représente un radical alkyle ou hydroxyalkyle ayant de 1 à 4 atomes de carbone et [Z] représente un radical alkylé polyhydrohydroxylé ayant de 3 à 10 atomes de carbone et de 3 à 10 groupes hydroxyle, ainsi qu'éventuellement
c) d'autres agents tensioactifs anioniques, non ioniques, amphotères ou Zwitterioniques et/ou des polymères en vue de la production de préparations de colorants et de pigments.

2. Utilisation d'agents dispersants selon la revendication 1,
caractérisée en ce qu'
on met en oeuvre des agents tensioactifs qui sont choisis dans le groupe formé par les alkylbenzène sulfonates, les alkanes sulfonates, les oléfine sulfonates, les alkyl éther sulfonates, les glycérol-éther sulfonates, les α-méthyle esters sulfonates, les acides gras sulfonés, les sulfates d'alkyle, les éthers sulfates d'alcool gras, les éthers sulfates de glycérol, les éthers sulfates mixtes hydroxylés, les monoglycéride (éther) sulfates, les éther sulfates d'amide d'acide gras, les sulfosuccinates, les sulfosuccinamates, les sulfotriglycérides, les acides éthercarboxyliques, les alkyloligoglucoside sulfatés et les alkyl (éther) phosphates.

3. Utilisation d'agents dispersants selon les revendications 1 et 2,
caractérisée en ce qu'
on met en oeuvre des agents tensioactifs non ioniques qui sont choisis dans le groupe formé par les éthers d'alcool gras et de polyglycol, les éthers d'alkylphénol et de polyglycol, les esters d'acide gras et de polyglycol, les éthers d'amide d'acide gras et de polyglycol, les éthers d'amide d'acide gras et de polyglycol, les esters d'amide et de polyglycol, les triglycérides alkoxylés, les esters d'acide gras et de polyol, les esters de sucre, les esters de sorbitanne et les polysorbates.

4. Utilisation d'agents dispersants selon les revendications 1 à 3,
caractérisée en ce qu'
on met en oeuvre des agents tensioactifs amphotères ou zwitterioniques qui sont choisis dans le groupe formé des alkylamidobétaïnes, des aminopropionates, des aminoglycinates, des bétaïnes d'imidazolinium et des sulfobétaïnes.

5. Utilisation d'agents dispersants selon les revendications 1 à 4,
caractérisée en ce qu'
on met en oeuvre en outre des condensats sulfonés du formaldéhyde avec le phénol ou le naphtalène.
